# EUROPEAN PATENT SPECIFICATION

(11) **EP 3 077 098 B1**
(45) Date of publication and mention of the grant of the patent: **07.03.2018**
(21) Application number: 14815535.1
(22) Date of filing: 01.12.2014
(51) Int. Cl.: B01J 13/04, C09D 101/08, C08L 1/08

(54) **PROCESS FOR PREPARING A MIXTURE OF A CELLULOSE DERIVATIVE AND A LIQUID DILUENT**
VERFAHREN ZUR HERSTELLUNG EINES GEMISCHS AUS EINEM CELLULOSEDERIVAT UND EINER VERDÜNNUNGSFLÜSSIGKEIT
PROCÉDÉ DE PRÉPARATION D'UN MÉLANGE D'UN DÉRIVÉ DE CELLULOSE ET UN DILUANT LIQUIDE

(30) Priority: 04.12.2013 US 201361911607 P
(43) Date of publication of application: 12.10.2016
(73) Proprietor: Dow Global Technologies LLC, Midland, MI 48674 (US)
(72) Inventor: HOELZER, Bettina, 89155 Erbach (DE); HILD, Alexandra, 29614 Soltau (DE); THEUERKAUF, Jorg, Lake Jackson, TX 77566 (US); HERMANNS, Juergen, 21640 Nottensdorf (DE)
(74) Representative: f & e patent
(86) International application number: PCT/US2014/067851
(87) International publication number: WO 2015/084696

(56) References cited:
- EP-A1- 1 338 288
- EP-A2- 2 454 955
- WO-A1-2008/050209
- DATABASE WPI Week 200862 Thomson Scientific, London, GB; AN 2008-K32970 XP002736258, & JP 2008 179918 A (TORAY IND INC) 7 August 2008 (2008-08-07)

## Description

### FIELD

The present invention concerns an improved process for preparing a mixture of a cellulose derivative, a process for the manufacture of capsules and a process for coating a dosage form with the mixture and the use of a cellulose derivative to reduce the time for at least partially removing entrapped air in said mixture.

### BACKGROUND

Cellulose derivatives, such as cellulose ethers and esters, are industrially important and are used in a large variety of technology areas and in many different end-use applications, for example in the personal care or pharmaceutical industry, in agricultural applications, or in the building or oil industry. Their preparation, properties and applications are described, for example, in Ullmann's Encyclopedia of Industrial Chemistry, 5th Edition, (1986), Volume A5, pages 461-488, VCH Verlagsgesellschaft, Weinheim or in "Methoden der organischen Chemie" (methods of organic chemistry), 4th Edition (1987), Volume E20, Makromolekulare Stoffe, Part Volume 3, pages 2048-2076, Georg Thieme Verlag, Stuttgart. Water-soluble cellulose ethers are conveniently supplied as a particulate dry material that is then dissolved in water for the desired end use.

It is well-known that water-soluble cellulose ethers tend to form "lumps" in water. Therefore, the blend of water-soluble cellulose ether and water is thoroughly stirred during the dissolution process. However, thoroughly stirred cellulose ether solutions tend to foaming caused by the gas, specifically air that is introduced into the cellulose ether solutions due to stirring. In industrial applications it is known to use a defoamer to reduce or avoid foaming. U.S. Patent No. 7,371,279 discloses a cellulose ether composition comprising certain cellulose ether, a superplasticizer and a defoamer. However, the use of a defoamer is not desirable for cellulose ethers which are used in pharmaceutical or food compositions. The inability to use a defoamer is particularly disadvantageous in applications where the cellulose ethers are used as film-forming material, such as in coatings or capsules. For such applications the cellulose ether solution has to be essentially free of gas bubbles, otherwise the films produced from these solutions have defects.

Similar problems can occur with other cellulose derivatives, such as cellulose esters or esterified cellulose ethers.

Accordingly, it is standing practice in the industry to let mixtures of cellulose derivatives with liquid diluents stand for a long time, e.g. for 24 hours or even longer to degas the mixtures. This significantly delays the production process and increases its costs. Applying a vacuum expedites the degassing process somewhat, but even at reduced pressure degassing of mixtures of cellulose derivatives and liquid diluents, particularly of aqueous solutions of cellulose ethers, takes a long time. Moreover, applying a vacuum requires expensive equipment.

Therefore, there is a strong need for an improved process for preparing a mixture of a cellulose derivative and a liquid diluent which does not require an unduly long time for degassing and/or the use of defoamer.

### SUMMARY

One aspect of the present invention is a process for preparing a mixture of a cellulose derivative and a liquid diluent comprising at least 5 weight percent of the cellulose derivative, based on the total weight of the cellulose derivative and the liquid diluent, wherein a cellulose derivative is provided which has a specific surface area of less than 0.20 m²/g, measured by BET method, and the cellulose derivative having said specific surface area is mixed with the liquid diluent.

Another aspect of the present invention is a process for manufacturing capsules which comprises the steps of preparing a mixture according to the above-mentioned process and contacting the mixture with dipping pins.

Yet another aspect of the present invention is a process for coating a dosage form which comprises the steps of preparing a mixture according to the above-mentioned process and contacting the mixture with the dosage form.

Yet another aspect of the present invention is the use of a cellulose derivative having a specific surface area of less than 0.20 m²/g measured by the BET method as disclosed in claim 13. Surprisingly, it has been found that the degassing time of a mixture of a cellulose derivative and a liquid diluent is significantly shorter when the mixture is prepared using a cellulose derivative that has a specific surface area of less than 0.20 m²/g than when a cellulose derivative having a larger specific surface area is chosen for preparing the mixture.

### DESCRIPTION OF EMBODIMENTS

The cellulose derivative utilized in the process of the present invention has a specific surface area of less than 0.20 m²/g, preferably from 0.02 to 0.18 m²/g, more preferably from 0.04 to 0.16 m²/g, even more preferably from 0.06 to 0.14 m²/g, and most preferably from 0.07 to 0.14 m²/g, measured by BET method. "BET" refers to the Brunauer-Emmett-Teller (BET) theory that aims to explain the physical adsorption of gas molecules on a solid surface and serves as the basis for an important analysis technique for the measurement of the specific surface area of a material. For the purpose of the present invention, the BET method is conducted according to DIN ISO 9277:2003-05.

Surprisingly, it has been found that the size and shapes of the cellulose derivative are not the important parameters for achieving a shortened degassing time. On the contrary, when the cellulose derivative utilized in the process of the present invention has a specific surface area of less than 0.20 m²/g, a shortened degassing time is achieved for cellulose derivatives having a wide range of particle sizes and shapes. Particle size and shape of a particulate cellulose derivative can be determined by a high speed image analysis method which combines particle size and shape analysis of sample images. An image analysis method for complex powders is described in: W. Witt, U. Kohler, J. List, Current Limits of Particle Size and Shape Analysis with High Speed Image Analysis, PARTEC 2007. A high speed image analysis system is commercially available from Sympatec GmbH, Clausthal-Zellerfeld, Germany as dynamic image analysis (DIA) system QICPIC™. The high speed image analysis system is useful for measuring among others the following dimensional parameters of particles:
EQPC: EQPC of a particle is defined as the diameter of a circle that has the same area as the projection area of the particle. The median EQPC is defined herein as the volume distribution average of all particles in a given sample of a particulate cellulose derivative. The median EQPC means that 50% of the EQPC of the particle distribution is smaller than the given value in µm and 50% is larger; it is designated herein as EQPC 50.3. The particulate cellulose derivative generally has a median EQPC of from 30 to 600 micrometers, typically from 100 to 500 micrometers, more typically from 150 to 450 micrometers.
LEFI: The particle length LEFI is defined as the longest direct path that connects the ends of the particle within the contour of the particle. "Direct" means without loops or branches. The median LEFI is defined herein as the volume distribution average of all particles in a given sample of a particulate cellulose derivative. The median LEFI means that 50% of the LEFI of the particle distribution is smaller than the given value in µm and 50% is larger; it is designated herein as LEFI 50.3. The particulate cellulose derivative generally has a median LEFI of from 50 to 1000 micrometers, typically from 100 to 850 micrometers, and more typically from 200 to 700 micrometers.

The cellulose derivatives used in this process are generally soluble or at least soakable in solvents, preferably water. They can have one or more substituents, preferably of the types: hydroxyethyl, hydroxypropyl, hydroxybutyl, methyl, ethyl, propyl, dihydroxypropyl, carboxymethyl, sulfoethyl, hydrophobic long-chain branched and unbranched alkyl groups, hydrophobic long-chain branched and unbranched alkyl aryl groups or aryl alkyl groups, cationic groups, acetate, propionate, butyrate, lactate, nitrate or sulfate, of which some groups, such as, for example, hydroxyethyl, hydroxypropyl, hydroxybutyl, dihydroxypropyl and lactate, are capable of forming grafts.

Preferred cellulose derivatives are cellulose esters or cellulose ethers. Useful cellulose ethers are, for example, carboxy-C₁-C₃-alkyl celluloses, such as carboxymethyl celluloses; carboxy-C₁-C₃-alkyl hydroxy-C₁-C₃-alkyl celluloses, such as carboxymethyl hydroxyethyl celluloses.

The cellulose ethers preferably are alkyl cellulose, hydroxyalkyl cellulose or hydroxyalkyl alkylcellulose. In such cellulose ethers at least a part of the hydroxyl groups of the anhydroglucose units are substituted by alkoxyl groups or hydroxyalkoxyl groups or a combination of alkoxyl and hydroxyalkoxyl groups. Typically one or two kinds of hydroxyalkoxyl groups are present in the cellulose ether. Preferably a single kind of hydroxyalkoxyl group, more preferably hydroxypropoxyl, is present.

Preferred alkyl celluloses are methylcelluloses or ethylcelluloses, more preferably methylcelluloses.

Preferred hydroxyalkyl celluloses are hydroxy-C₂₋₄-alkyl celluloses, such as hydroxybutyl celluloses, hydroxypropyl cellulose or, preferably, hydroxyethyl cellulose, or mixed hydroxylkyl celluloses, such as hydroxyethyl hydroxypropyl celluloses.

Preferred alkyl hydroxyalkyl celluloses including mixed alkyl hydroxyalkyl celluloses are hydroxyalkyl methylcelluloses, such as hydroxyethyl methylcelluloses, hydroxypropyl methylcelluloses or hydroxybutyl methylcelluloses; or hydroxyalkyl ethyl celluloses, such as hydroxypropyl ethylcelluloses, ethyl hydroxyethyl celluloses, ethyl hydroxypropyl celluloses or ethyl hydroxybutyl celluloses; or ethyl hydroxypropyl methylcelluloses, ethyl hydroxyethyl methylcelluloses, hydroxyethyl hydroxypropyl methylcelluloses or alkoxy hydroxyethyl hydroxypropyl celluloses, the alkoxy group being straight-chain or branched and containing 2 to 8 carbon atoms.

Preferred are hydroxyalkyl alkylcelluloses, more preferred are hydroxyalkyl methylcelluloses and most preferred are hydroxypropyl methylcelluloses, which have an MS(hydroxyalkoxyl) and a DS(alkoxyl) described below.

The cellulose derivatives are preferably water-soluble, i.e., they have a solubility in water of at least 1 gram, more preferably at least 2 grams, and most preferably at least 5 grams in 100 grams of distilled water at 25 °C and 1 atmosphere.

The degree of the substitution of hydroxyl groups of the anhydroglucose units by hydroxyalkoxyl groups is expressed by the molar substitution of hydroxyalkoxyl groups, the MS(hydroxyalkoxyl). The MS(hydroxyalkoxyl) is the average number of moles of hydroxyalkoxyl groups per anhydroglucose unit in the cellulose ether. It is to be understood that during the hydroxyalkylation reaction the hydroxyl group of a hydroxyalkoxyl group bound to the cellulose backbone can be further etherified by an alkylation agent, e.g. a methylation agent, and/or a hydroxyalkylation agent. Multiple subsequent hydroxyalkylation etherification reactions with respect to the same carbon atom position of an anhydroglucose unit yields a side chain, wherein multiple hydroxyalkoxyl groups are covalently bound to each other by ether bonds, each side chain as a whole forming a hydroxyalkoxyl substituent to the cellulose backbone. The term "hydroxyalkoxyl groups" thus has to be interpreted in the context of the MS(hydroxyalkoxyl) as referring to the hydroxyalkoxyl groups as the constituting units of hydroxyalkoxyl substituents, which either comprise a single hydroxyalkoxyl group or a side chain as outlined above, wherein two or more hydroxyalkoxyl units are covalently bound to each other by ether bonding. Within this definition it is not important whether the terminal hydroxyl group of a hydroxyalkoxyl substituent is further alkylated, e.g. methylated, or not; both alkylated and non-alkylated hydroxyalkoxyl substituents are included for the determination of MS(hydroxyalkoxyl).

The hydroxyalkyl alkylcellulose generally has a molar substitution of hydroxyalkoxyl groups in the range of 0.05 to 1.00, preferably 0.08 to 0.90, more preferably 0.12 to 0.70, most preferably 0.15 to 0.60, and particularly 0.20 to 0.50.

The average number of hydroxyl groups substituted by alkoxyl groups, such as methoxyl groups, per anhydroglucose unit, is designated as the degree of substitution of alkoxyl groups, DS(alkoxyl). In the above-given definition of DS, the term "hydroxyl groups substituted by alkoxyl groups" is to be construed within the present invention to include not only alkylated hydroxyl groups directly bound to the carbon atoms of the cellulose backbone, but also alkylated hydroxyl groups of hydroxyalkoxyl substituents bound to the cellulose backbone. The hydroxyalkyl alkylcelluloses according to this invention preferably have a DS(alkoxyl) in the range of 1.0 to 2.5, more preferably 1.1 to 2.4, most preferably 1.2 to 2.2 and particularly 1.6 to 2.05. Most preferably the cellulose ether is a hydroxypropyl methylcellulose or hydroxyethyl methylcellulose having a DS(methoxyl) within the ranges indicated above for DS(alkoxyl) and an MS(hydroxypropoxyl) or an MS(hydroxyethoxyl) within the ranges indicated above for MS(hydroxyalkoxyl). The degree of substitution of alkoxyl groups and the molar substitution of hydroxyalkoxyl groups can be determined by Zeisel cleavage of the cellulose ether with hydrogen iodide and subsequent quantitative gas chromatographic analysis (G. Bartelmus and R. Ketterer, Z. Anal. Chem., 286 (1977) 161-190).

Preferred alkyl celluloses are methylcelluloses. The average number of hydroxyl groups substituted by methoxyl groups per anhydroglucose unit is designated as the degree of substitution of methoxyl groups (DS). The methylcellulose preferably has a DS of from 1.20 to 2.25, more preferably from 1.25 to 2.20, and most preferably from 1.40 to 2.10. The determination of the % methoxyl in methylcellulose is carried out according to the United States Pharmacopeia (USP 34). The values obtained are % methoxyl. These are subsequently converted into degree of substitution (DS) for methoxyl substituents.

The viscosity of the cellulose derivative is generally from 1.2 to 200 mPa·s, preferably from 2 to 100 mPa·s, more preferably from 2.5 to 50 mPa·s, and in particular from 3 to 30 mPa·s, measured as a 2.0 weight-% aqueous solution at 20 °C. The 2.0 % by weight HPMC solution in water is prepared according to United States Pharmacopeia (USP 35, "Hypromellose", pages 3467-3469) followed by an Ubbelohde viscosity measurement according to DIN 51562-1:1999-01 (January 1999).

According to the present invention an above-described cellulose derivative is mixed with a liquid diluent to produce a mixture that comprises at least 5 weight percent, preferably at least 7 weight percent, and more preferably at least 10 weight percent of the cellulose derivative, based on the total weight of the cellulose derivative and the liquid diluent. Typically the above-described cellulose derivative is mixed with a liquid diluent to produce a mixture that comprises up to 40 weight percent, typically up to 30 weight percent, and more typically up to 25 weight percent of the cellulose derivative, based on the total weight of the cellulose derivative and the liquid diluent. More than one type of an above-described cellulose derivative can be mixed with a liquid diluent, however, the total amount of cellulose derivatives should be within the weight percentages described above. "Liquid diluent" as used herein means a diluent that is liquid a 20 °C and atmospheric pressure.

When the cellulose derivative(s) is/are water-soluble, preferably an aqueous liquid diluent is used, i.e. a diluent which has a water content of more than 50 weight percent and up to 100 percent of water. The aqueous liquid may additionally comprise a minor amount of an organic liquid diluent; however, the aqueous liquid generally comprises more than 50, preferably at least 65, more preferably at least 75, most preferably at least 90, and particularly at least 95 weight percent of water, based on the total weight of the aqueous liquid. Preferably the aqueous liquid consists of water.

On the other hand, when the cellulose derivative(s) is/are water-insoluble, i.e., when it has /they have a solubility in water of at less than 1 gram in 100 grams of distilled water at 25 °C and 1 atmosphere, preferably an organic liquid diluent is used for preparing the mixture. The term an "organic liquid diluent" as used herein means an organic solvent or a mixture of two or more organic solvents. Preferred organic liquid diluents are polar organic solvents having one or more heteroatoms, such as oxygen, nitrogen or halogen like chlorine. More preferred organic liquid diluents are alcohols, for example multifunctional alcohols, such as glycerol, or preferably monofunctional alcohols, such as methanol, ethanol, isopropanol or n-propanol; ethers, such as tetrahydrofuran, ketones, such as acetone, methyl ethyl ketone, or methyl isobutyl ketone; acetates, such as ethyl acetate; halogenated hydrocarbons, such as methylene chloride; or nitriles, such as acetonitrile. More preferably the organic liquid diluents have 1 to 6, most preferably 1 to 4 carbon atoms. The organic diluent may be used alone or mixed with a minor amount of water. In this embodiment of the present invention the liquid diluent preferably comprises more than 50, more preferably at least 65, and most preferably at least 75 weight percent of an organic liquid diluent and preferably less than 50, more preferably up to 35, and most preferably up to 25 weight percent of water, based on the total weight of the organic liquid diluent and water.

In the mixing operation a dispersion or preferably a solution of the cellulose derivative in the liquid diluent is prepared. The mixture is typically produced by bringing the cellulose derivative and optional additives into contact with the liquid diluent under thorough stirring using known agitation devices, such as known stirrers, to minimize or avoid the formation of lumps. Optional additives may be incorporated into the mixture, such as coloring agents, flavor and taste improvers, antioxidants, plasticizers or a combination thereof. For example, when the mixture is intended for producing capsules a water-soluble food dye, such as red oxide, or a natural dye, may be used as a coloring agent; TiO₂ may be used as a masking agent; sorbitol or glycerin may be used as a plasticizer to improve the flexibility of the capsule film. Particularly useful additives for coatings of solid dosage forms are single layer film plasticizers, solids-loading enhancers, a second cellulose derivative, preferably a second cellulose ether, lubricants, polishing agents, pigments, anti-tack agents, glidants, opacifiers, coloring agents and any combination thereof.

The mixture is preferably prepared in the absence of a substantial amount of a defoaming agent. Typically the mixture comprises less than 0.15 wt.%, more typically less than 0.1 wt.%, even more typically less than 0.05 wt.%, and most typically less than 0.02 wt.%, based on the weight of the cellulose derivative. Known defoaming agents are listed in U.S. Patent No. 7,371,279, e.g., alkylene glycol homopolymers, copolymers, terpolymers and block copolymers, for example based on ethylene oxide and propylene oxide, adducts of alkylene oxides, alkylene glycol ethers of higher alcohols, alkylene glycol fatty acid esters, sorbitol fatty acid esters, polyoxyalkylene sorbitol fatty acid esters, addition products of ethylene oxide and propylene oxide and acetylene, phosphate esters such as tributyl phosphate or sodium octyl phosphate and polyether-containing compounds and polyether-containing mixtures having defoamer action.

The mixing operating, particularly mixing at high tip speed of the mixing device, causes air to be entrapped in the mixture. Accordingly, after the mixture has been produced, it is allowed to stand at or below atmospheric pressure, preferably at atmospheric pressure, to reduce the volume of air entrapped in the mixture. When the mixture is left to stand below atmospheric pressure, the pressure is typically 5 to 500 millibar, more typically 10 to 100 millibar. Typically the mixture is left to stand at a temperature of 5 - 35 °C, more typically of 15 - 25 °C. How long the mixture is allowed to stand depends on various factors, such as the pressure at which the mixture is allowed to stand, the temperature, the volume of air entrapped in the mixture and the extent to which the entrapped air should be removed. However, the mixture is typically allowed to stand for 0.5 to 24 hours, more typically for 1 to 18 hours, and most typically for 2 to 12 hours.

Surprisingly, it has been found that the time for at least partially removing entrapped air from a prepared mixture of cellulose derivative and liquid diluent is significantly reduced if a cellulose derivative having a specific surface area of less than 0.20 m²/g, measured by BET method, is chosen for preparing the mixture. The time for at least partially removing entrapped air from the mixture is reduced even if the cellulose derivative has substantially the same substituents, degrees of substitution, viscosity, average particle length and average particle diameter as a cellulose derivative having a specific surface area of more than 0.20 m²/g measured by BET method. E.g., the reduced time for at least partially removing entrapped air from the mixture is shown by the fact that after storage of the mixture for a given time period, e.g., for 4, 6 or 8 hours, a larger percentage of the volume of the mixture is clear, indicating the essential absence of entrapped air, when a cellulose derivative having a specific surface area of less than 0.20 m²/g has been used for preparing the mixture than when using a corresponding cellulose derivative having a specific surface area of more than 0.20 m²/g.

The mixture prepared according to the above-described process may be used for coating dosage forms, such as tablets, granules, pellets, caplets, lozenges, suppositories, pessaries or implantable dosage forms, to form a coated dosage form. Preferred dosage forms are pharmaceutical dosage forms, nutrition supplements or agricultural dosage forms.

In another aspect of the invention the mixture prepared according to the above-described process may be used for the manufacture of capsules. A known method for the manufacture of capsules is the "hot-pin method", such as described in detail in the International Patent Publication No. WO 2008/050209. Another known method for the manufacture of capsules is the "cold-pin method", such as described in detail in European Patent Application No. EP 0 714 656 and in US Patent No. 6,410,050.

Some embodiments of the invention will now be described in detail in the following Examples.

### EXAMPLES

Unless otherwise mentioned, all parts and percentages are by weight. In the Examples the following test procedures are used.

### BET method of hydroxypropyl methylcellulose (HPMC)

The specific surface area of the cellulose derivative particles, specifically HPMC, was measured by the BET method. The BET method was conducted according to DIN ISO 9277:2003-05. The Krypton BET was measured using a Micromeritics ASAP 2020 instrument after complete drying (7 h at 105°C).

### Viscosity of HPMC

The viscosity of the HPMC samples was measured as a 2.0 % by weight solution in water at 20°C. The 2.0 % by weight HPMC solution in water was prepared according to United States Pharmacopeia (USP 35, "Hypromellose", pages 3467-3469) followed by an Ubbelohde viscosity measurement according to DIN 51562-1:1999-01 (January 1999).

### LEFI and EQPC of HPMC

The partizle sizes and shapes of the cellulose derivative particles were determined by a high speed image analysis method which combines particle size and shape analysis of sample images. An image analysis method for complex powders is described in: W. Witt, U. Kohler, J. List, Current Limits of Particle Size and Shape Analysis with High Speed Image Analysis, PARTEC 2007. A high speed image analysis system which is commercially available from Sympatec GmbH, Clausthal- Zellerfeld, Germany as dynamic image analysis (DIA) system QICPIC™ was used for the particle size and shape analysis. The high speed image analyzer sensor QICPIC, Sympatec, Germany was used in combination with a dry disperser RODOS/L with an inner diameter of 4 mm and a dry feeder VIBRI/L and Software WINDOX5, Vers. 5.3.0 and M7 lens).

The following dimensional parameters of the particles were measured and listed in Table 1 below.

EQPC: EQPC of a particle is defined as the diameter of a circle that has the same area as the projection area of the particle. The median EQPC is defined herein as the volume distribution average of all particles in a given sample of a particulate cellulose derivative. The median EQPC means that 50% of the EQPC of the particle distribution is smaller than the given value in µm and 50% is larger; it is designated herein as EQPC 50.3.

LEFI: The particle length LEFI is defined as the longest direct path that connects the ends of the particle within the contour of the particle. "Direct" means without loops or branches. The median LEFI is defined herein as the volume distribution average of all particles in a given sample of a particulate cellulose derivative. The median LEFI means that 50% of the LEFI of the particle distribution is smaller than the given value in µm and 50% is larger; it is designated herein as LEFI 50.3.

### HPMC Preparation

### Comparative Example A

A HPMC which was commercially available from The Dow Chemical Company as METHOCEL™ F4M and which had 29.2 % methoxyl groups, 6.2 % hydroxypropoxyl groups and a viscosity of 5200 mPa·s, measured as a 2.0 % solution in water at 20°C, was used as a starting material for producing the HPMC of Comparative Example A. The HPMC was partially depolymerized by heating the powderous samples with about 0.2 % gaseous hydrogen chloride, based on the weight of the HPMC, at a temperature of 80 - 85 °C for 55 - 65 minutes to produce a HPMC of 4 - 6 mPa·s. The produced partially depolymerized HPMC had 29.2 % methoxyl groups, 6.2 % hydroxypropoxyl groups and a viscosity of 5.2 mPa·s, measured as a 2.0 % by weight solution in water at 20°C.

### Comparative Example B and Example 1

To 250 g of the HPMC of Comparative Example A 293 g deionized water were added in 15 min at room temperature under stirring in a lab granulator (Bosch, ProfiMixx 44 with dough hook) resulting in 55 % moisture of the product. After additional granulation for 30 min at room temperature the product was dried overnight at 55 °C in a drying oven with recirculating air. The product was milled using an Alpine mill (Alpine lab mill 100 UPZ II with a 0.5 µm milling sieve, premilling was conducted using Erweka AR401/TG2000) and subsequent sieved using a 500, 250 and 63 µm sieve. The specific surfaces of the sieve fractions were measured.

The sieve fraction which passed through the 500 µm sieve but not through the 250 µm sieve had a specific surface area of less than 0.20 m²/g, measured by BET method and is designated as Example 1 in Table 1 below.

The sieve fraction which passed through the 250 µm sieve but not through the 63 µm sieve had a specific surface area of more than 0.20 m²/g, measured by BET method and is designated as Comparative Example B in Table 1 below.

### Example 2

A HPMC which had 29.2 % methoxyl groups, 6.3 % hydroxypropoxyl groups and a viscosity of about 4000 mPa·s, measured as a 2.0 % solution in water at 20°C, was used as a starting material for producing the HPMC particles of Example 2. The production of the HPMC particles involved the compounding of the HPMC with water, milling and drying the mixture in an impact mill and partial depolymerization of the resulting HPMC particles as described below.

The HPMC having a temperature of 20 °C was fed continuously at a feed rate of 20 kg/h into a commercially available continuous compounder with heating and cooling jacket. Water of a temperature of 5 °C was continuously added to the compounder to achieve a moisture of 73%, based on the total weight of the moist HPMC. The moist HPMC product was transported continuously via a transport belt into a mill feed unit (Altenburger Maschinen Jaeckering GmbH, Hamm, Germany). The mill feed unit was a vessel equipped with a vessel agitator having blades and a single auger screw. The bottom blades of the vessel agitator pressed the paste into a single augur screw mounted at the bottom of the vessel.

The wet product was forced through a perforated plate directly into the side of an Ultrarotor II "S" impact mill (Altenburger Maschinen Jaeckering GmbH, Hamm, Germany) between the first and second grinding stage. The impact mill was equipped with seven grinding stages. The bottom five grinding stages were equipped with standard grinding bars. No grinding bars were installed in the top two grinding stages. The interior of mill jacket had the standard Altenburger corrugated stationary grinding plates. The rotor of the impact mill was operated at a circumferential speed of 87 m/s. A specific closed loop gas flow system applying nitrogen as gas was used as carrier and drying gas. The gas flow system was composed of three separately controllable gas streams. One gas stream was designed to flow through the impact mill. A second gas stream was designed to flow through a by-pass. The HPMC leaving the impact mill was contacted with this second gas stream for drying purposes. A third gas stream was cooled and was used for temperature control. The control operation was carried out by slide valves allowing controlling the amount of the respective gas stream. At the same time the temperature of the gas streams could be controlled via a natural gas burner and a gas cooling system using cold water as coolant. The gas flow system is described in detail in the International Patent Application WO 2012/138533, specifically in Figure 1 and the description thereof. The lay-out of the gas flow system was as described in Table 1 of WO 2012/138533, except that different conditions were applied in the process to obtain HPMC particles having a specific surface area of less than 0.20 m²/g, measured by BET method.

The conditions in the present Example 2 are listed in Table 1 below.

The resulting HPMC particles were not sieved. They were partially depolymerized by heating the HPMC particles with about 0.2 % gaseous hydrogen chloride, based on the weight of the HPMC, at a temperature of 80 - 85 °C for 55 - 65 minutes to produce a HPMC of 4 - 6 mPa·s. The produced partially depolymerized HPMC had 29.2 % methoxyl groups, 6.3 % hydroxypropoxyl groups and a viscosity of 4.6 mPa·s, measured as a 2.0 % by weight solution in water at 20°C.

**Table 1**

| | |
|---|---|
| HPMC Moisture before grinding [%] | 73 |
| HPMC Temperature before grinding [°C] | 24 |
| Water Temperature [°C] | 5 |
| Total Gas stream [m³/h] | 1028 |
| Gas stream through bypass [m³/h] | 752 |
| Gas stream through mill [m³/h] | 275 |
| Cooled gas flow [m³/h] | 463 |
| Throughput HPMC, based on dry weight [kg/h] | 20 |
| Gas through mill / HPMC in Mill [m³/kg] | 14 |
| Gas through bypass / HPMC in Mill [m³/kg] | 38 |
| Circumferential Speed of mill [m/s] | 87 |
| Temp. of heated gas stream after burner and in by-pass [°C] | 137 |
| Temperature of gas stream before mill [°C] | 25 |
| Difference between temperature in by-pass and in gas stream before mill | 112 |
| Temperature gas stream after mill [°C] | 54 |
| Temperature of combined gas stream [°C] | 106 |
| Difference between temp. in gas stream after mill and in combined gas stream [°C] | 49 |
| Temperature gas stream before filter [°C] | 91 |
| Temperature gas stream before blower [°C] | 34 |
| Difference between gas temperature in bypass and gas temperature after mill [°C] | 112 |
| Final Moisture [%] | 4.0 |

### Determination of Degassing Time:

In all Examples and Comparative Examples a 20 % solution was prepared according to the following procedure:
To 200 g deionized water in a 1 L beaker at 20°C 50 g HPMC (absolute dry) were added in 30 seconds with subsequent stirring at 750 rpm using a propeller stirrer for 1.5 h at 20°C. The solutions were stored in a vacuum oven at 22 °C at 20 mbar for 20 hours. Every 10 min it was recorded in mm using a ruler how much clear solution on the bottom of the beaker was obtained and related to the complete amount of solution available. A clear solution indicated the essential absence of gas bubbles. In the presence of a substantial number of gas bubbles the solution was turbid.

**Table 2**

| | **Comp. Ex. A** | **Example 1** | **Comp. Ex. B** | **Example 2** |
|---|---|---|---|---|
| Specific surface, BET [m²/g] | 0.29 | 0.12 | 0.22 | 0.09 |
| Degassing after 4h, [%] | 31 | 52 | 34 | 56 |
| Degassing after 6h, [%] | 43 | 67 | 50 | 71 |
| Degassing after 8h, [%] | 59 | 83 | 63 | 89 |
| LEFI 50.3 [µm] | 248 | 612 | 283 | 302 |
| EQPC 50.3 [µm] | 84 | 402 | 184 | 193 |
| 2% viscosity [mPa·s] | 5 | 5 | 5 | 5 |

The results in Table 2 illustrate that aqueous solutions comprising the cellulose derivatives of Examples 1 and 2, which both have a specific surface area of less than 0.20 m²/g, measured by BET method, exhibit a considerably faster degassing time than the aqueous solutions comprising the cellulose derivatives of Comparative Examples A and B, which both have a specific surface area of more than 0.20 m²/g. After 4 hours storage in a vacuum oven at 22°C at 20 mbar, more than 50% of the volume of the solutions of Examples 1 and 2 was clear, whereas less than 35% of the volume of the solutions of Comparative Examples A and B was clear. For the solutions of Comparative Examples A and B 8 hours storage in a vacuum oven at 22°C at 20 mbar was needed to achieve that more than 50% of the volume of the solutions was clear.

The HPMCs of Examples 1 and 2 have a very similar specific surface and a similar degassing behavior, although they have been produced in a different manner and although the morphologies of the particles of the HPMCs of Examples 1 and 2 are very different. The median LEFI and EQPC in the HPMC of Example 1 are about twice as high as the median LEFI and EQPC in the HPMC of Example 2.

The HPMCs of Comparative Examples A and B have a specific surface of more than 0.20 m²/g. They both have a similar degassing behavior, although the particles of the granulated HPMC of Comparative Example B are much thicker than the HPMC particles of Comparative Example A, as evidenced by a much higher median EQPC.

The aqueous solution of the HPMC of Example 2 exhibits considerably faster degassing than the aqueous solution of the HPMC of Comparative Example B. The HPMC of Example 2 has a specific surface area of less than 0.20 m²/g, whereas the HPMC of Comparative Example B has a specific surface area of more than 0.20 m²/g. The HPMCs of Example 2 and of Comparative Example B have a similar median LEFI and EQPC. This illustrates that the specific surface of the cellulose derivative is decisive for the degassing behavior of the cellulose derivative after it has been mixed with a liquid diluent.

## Claims

1. A process for preparing a mixture of a cellulose derivative and a liquid diluent comprising at least 5 weight percent of the cellulose derivative, based on the total weight of the cellulose derivative and the liquid diluent, wherein a cellulose derivative is provided which has a specific surface area of less than 0.20 m²/g, measured by BET method, and the cellulose derivative having said specific surface area is mixed with the liquid diluent.

2. The process of claim 1, wherein a cellulose derivative having a specific surface area of from 0.04 to 0.16 m²/g is provided.

3. The process of claim 1 or 2, wherein the cellulose derivative has a viscosity of from 1.2 to 200 mPaxs, measured as a 2 weight-% aqueous solution at 20 °C according to ASTM D2363 - 79 Reapproved 2006.

4. The process of any one of claims 1 to 3 for preparing a mixture comprising from 7 to 30 weight percent of the cellulose derivative, based on the total weight of the cellulose derivative and the liquid diluent.

5. The process of any one of claims 1 to 4, wherein the cellulose derivative is mixed with an aqueous diluent.

6. The process of any one of claims 1 to 5, wherein the cellulose derivative is a cellulose ether or cellulose ester.

7. The process of claim 6, wherein the cellulose ether is a hydroxypropyl methylcellulose, a methylcellulose or a hydroxyethyl cellulose.

8. The process of any one of claims 1 to 7, wherein the mixture comprise less than 0.15 wt.%, of a defoaming agent based on the weight of the cellulose derivative.

9. The process of any one of claims 1 to 8, wherein the mixture is allowed to stand at or below atmospheric pressure after it has been produced.

10. The process of claim 9 wherein the mixture is allowed to stand at atmospheric pressure after it has been produced.

11. A process for manufacturing capsules comprising the steps of preparing a mixture according to the process of any one of claims 1 to 10 and contacting the mixture with dipping pins.

12. A process for coating a dosage form comprising the steps of preparing a mixture according to the process of any one of claims 1 to 10 and contacting the mixture with the dosage form.

13. Use of a cellulose derivative having a specific surface area of less than 0.20 m²/g measured by BET method in a process comprising the steps of
i) mixing the cellulose derivative with a liquid diluent to provide a mixture comprising at least 5 weight percent of cellulose derivative, based on the total weight of the cellulose derivative and the liquid diluent, wherein the mixing operating causes air to be entrapped in the mixture, and
ii) reducing the volume of air entrapped in the mixture by allowing the mixture to stand at or below atmospheric pressure,
to reduce the time for at least partially removing entrapped air.

14. The use of claim 13 wherein a cellulose derivative is provided that has a specific surface area of from 0.04 to 0.16 m²/g.

15. The use of claim 13 or 14 wherein the cellulose derivative is a cellulose ether or cellulose ester.

## Patentansprüche

1. Verfahren zur Herstellung einer Mischung eines Cellulosederivats und eines flüssigen Verdünnungsmittels, die wenigstens 5 Gew.-% des Cellulosederivats, bezogen auf das Gesamtgewicht von Cellulosederivat und flüssigem Verdünnungsmittel, enthält, wobei ein Cellulosederivat bereitgestellt wird mit einer spezifischen Oberfläche von weniger als 0,20 m²/g, gemessen nach der BET-Methode, und das Cellulosederivat mit dieser spezifischen Oberfläche mit dem flüssigen Verdünnungsmittel vermischt wird.

2. Verfahren nach Anspruch 1, wobei ein Cellulosderivat mit einer spezifischen Oberfläche von 0,04 bis 0,16 m²/g bereitgestellt wird.

3. Verfahren nach Anspruch 1 oder 2, wobei das Cellulosederivat eine Viskosität von 1,2 bis 200 mPa·s, gemessen als 2-gew.-%ige wässrige Lösung bei 20°C gemäß ASTM D2363 - 79 wiedergenehmigt 2006, aufweist.

4. Verfahren nach einem der Ansprüche 1 bis 3 zur Herstellung einer Mischung, die 7 bis 30 Gew.-% des Cellulosederivats, bezogen auf das Gesamtgewicht von Cellulosederivat und flüssigem Verdünnungsmittel, enthält.

5. Verfahren nach einem der Ansprüche 1 bis 4, wobei das Cellulosederivat mit einem wässrigen Verdünnungsmittel gemischt wird.

6. Verfahren nach einem der Ansprüche 1 bis 5, wobei das Cellulosederivat ein Celluloseether oder Celluloseester ist.

7. Verfahren nach Anspruch 6, wobei der Celluloseether eine Hydroxypropylmethylcellulose, eine Methylcellulose oder eine Hydroxyethylcellulose ist.

8. Verfahren nach einem der Ansprüche 1 bis 7, wobei die Mischung weniger als 0,15 Gew.-% eines Antischaummittels, bezogen auf das Gewicht des Cellulosederivats, enthält.

9. Verfahren nach einem der Ansprüche 1 bis 8, wobei die Mischung bei oder unterhalb atmosphärischem Druck stehen gelassen wird, nachdem sie hergestellt worden ist.

10. Verfahren nach Anspruch 9, wobei die Mischung bei atmosphärischem Druck stehen gelassen wird, nachdem sie hergestellt worden ist.

11. Verfahren zur Herstellung von Kapseln, umfassend den Schritt der Herstellung einer Mischung gemäß dem Verfahren nach einem der Ansprüche 1 bis 10 und In-Kontakt-Bringen der Mischung mit Tauchnadeln.

12. Verfahren zur Beschichtung einer Dosierform, umfassend die Schritte der Herstellung einer Mischung gemäß dem Verfahren nach einem der Ansprüche 1 bis 10 und In-Kontakt-Bringen der Mischung mit der Dosierform.

13. Verwendung eines Cellulosederivats mit einer spezifischen Oberfläche von weniger als 0,20 m²/g, gemessen mit der BET-Methode, in einem Verfahren, umfassend die Schritte von
(i) Mischen des Cellulosederivats mit einem flüssigen Verdünnungsmittel, um eine Mischung bereitzustellen, die wenigstens 5 Gew.-% Cellulosederivat enthält, bezogen auf das Gesamtgewicht des Cellulosederivats und des flüssigen Verdünnungsmittels, wobei der Mischvorgang dazu führt, dass Luft in die Mischung eingeschlossen wird, und
(ii) Verringern des eingeschlossenen Luftvolumens in der Mischung durch Stehenlassen der Mischung bei oder unterhalb von atmosphärischem Druck,
um die Zeit zur wenigstens teilweisen Entfernung der eingeschlossenen Luft zu verringern.

14. Verwendung nach Anspruch 13, wobei ein Cellulosederivat bereitgestellt wird, das eine spezifische Oberfläche von 0,04 bis 0,16 m²/g aufweist.

15. Verwendung nach Anspruch 13 oder 14, wobei das Cellulosederivat ein Celluloseether oder Celluloseester ist.

## Revendications

1. Un procédé pour la préparation d'un mélange d'un dérivé de cellulose et d'un diluant liquide comprenant au moins 5 pour cent en poids du dérivé de cellulose, rapporté au poids total du dérivé de cellulose et du diluant liquide, où il est fourni un dérivé de cellulose qui présente une aire spécifique de moins de 0,20 m²/g, mesurée par la méthode BET, et le dérivé de cellulose présentant ladite aire spécifique est mélangé avec le diluant liquide.

2. Le procédé de la revendication 1, où il est fourni un dérivé de cellulose présentant une aire spécifique allant de 0,04 à 0,16 m²/g.

3. Le procédé de la revendication 1 ou de la revendication 2, où le dérivé de cellulose présente une viscosité allant de 1,2 à 200 mPa×s, mesurée sous forme de solution aqueuse à 2 % en poids à 20 °C selon l'ASTM D2363 - 79 approuvé de nouveau en 2006.

4. Le procédé de l'une quelconque des revendications 1 à 3 pour la préparation d'un mélange comprenant de 7 à 30 pour cent en poids du dérivé de cellulose, rapporté au poids total du dérivé de cellulose et du diluant liquide.

5. Le procédé de l'une quelconque des revendications 1 à 4, où le dérivé de cellulose est mélangé avec un diluant aqueux.

6. Le procédé de l'une quelconque des revendications 1 à 5, où le dérivé de cellulose est un éther de cellulose ou ester de cellulose.

7. Le procédé de la revendication 6, où l'éther de cellulose est une hydroxypropyl méthylcellulose, une méthylcellulose ou une hydroxyéthyl cellulose.

8. Le procédé de l'une quelconque des revendications 1 à 7, où le mélange comprend moins de 0,15 % en poids d'un agent antimousse rapporté au poids du dérivé de cellulose.

9. Le procédé de l'une quelconque des revendications 1 à 8, où le mélange est laissé à reposer à ou en dessous de la pression atmosphérique une fois produit.

10. Le procédé de la revendication 9 où le mélange est laissé à reposer à la pression atmosphérique une fois produit.

11. Un procédé pour la fabrication de capsules comprenant les étapes consistant à préparer un mélange selon le procédé de l'une quelconque des revendications 1 à 10 et à mettre le mélange en contact avec des tiges d'immersion.

12. Un procédé pour l'enrobage d'une forme galénique comprenant les étapes consistant à préparer un mélange selon le procédé de l'une quelconque des revendications 1 à 10 et à mettre le mélange en contact avec la forme galénique.

13. Utilisation d'un dérivé de cellulose présentant une aire spécifique de moins de 0,20 m²/g mesurée par la méthode BET dans un procédé comprenant les étapes consistant
i) à mélanger le dérivé de cellulose avec un diluant liquide afin de fournir un mélange comprenant au moins 5 pour cent en poids de dérivé de cellulose, rapporté au poids total du dérivé de cellulose et du diluant liquide, où l'opération de mélange provoque l'inclusion d'air dans le mélange, et
ii) à réduire le volume d'air inclus dans le mélange en laissant le mélange reposer à ou en dessous de la pression atmosphérique,
afin de réduire le temps pour chasser au moins partiellement l'air inclus.

14. L'utilisation de la revendication 13 où il est fourni un dérivé de cellulose qui présente une aire spécifique allant de 0,04 à 0,16 m²/g.

15. L'utilisation de la revendication 13 ou de la revendication 14 où le dérivé de cellulose est un éther de cellulose ou ester de cellulose.
